# FASCICULE DE BREVET EUROPEEN

(11) **EP 2 200 670 B1**
(45) Date de publication et mention de la délivrance du brevet: **28.12.2011**
(21) Numéro de dépôt: 08841581.5
(22) Date de dépôt: 22.10.2008
(51) Int. Cl.: A61L 27/12, A61L 27/50, A61K 6/00, A61K 49/04, A61K 49/06, C04B 12/02

(54) **SUBSTITUT OSSEUX COMPRENANT UN AGENT DE CONTRASTE, SON PROCEDE DE PREPARATION ET SES UTILISATIONS**
KNOCHENERSATZ MIT KONTRASTMITTEL, VERFAHREN ZU SEINER HERSTELLUNG UND VERWENDUNG
BONE SUBSTITUTE CONTAINING A CONTRAST AGENT, METHOD FOR PREPARING SAME AND USES THEREOF

(30) Priorité: 22.10.2007 FR 0758464
(43) Date de publication de la demande: 30.06.2010
(73) Titulaire: Biomatlante, 44360 Vigneux de Bretagne (FR)
(72) Inventeur: BOURGES, Xavier, 01140 Mogneneins (FR); BAROTH, Serge, 44400 Nantes (FR); DACULSI, Guy, F-44360 Vigneux de Bretagne (FR)
(74) Mandataire: Ahner, Francis
(86) Numéro de dépôt international: PCT/EP2008/064229
(87) Numéro de publication internationale: WO 2009/053372

(56) Documents cités:
- WO-A-93/07905
- WO-A-95/21634
- FR-A- 2 857 268
- DATABASE WPI Week 200776 Thomson Scientific, London, GB; AN 2007-809074 XP002481167 -& JP 2007 197329 A (KURARE MEDICAL KK) 9 août 2007 (2007-08-09)

## Description

La présente invention se rapporte à un biomatériau de comblement osseux utilisable en chirurgie, qui comprend un agent de contraste permettant au chirurgien de contrôler par imagerie médicale le succès de l'implantation dudit biomatériau ainsi que sa résorption et substitution par de l'os nouvellement formé.

Les mélanges d'hydroxyapatite et de phosphate tricalcique β (β-TCP) constituent la base minérale de nombreux matériaux de comblement utiles en chirurgie osseuse. Ils permettent d'associer la stabilité de l'hydroxyapatite, qui constitue un support d'adhésion efficace pour les ostéoblastes, et la bonne résorption du β-TCP, grâce à la libération d'ions calcium par ce dernier.

Les matrices d'hydroxyapatite et de β-TCP peuvent être utilisées efficacement dans tout type de comblement osseux, notamment en chirurgie orthopédique, cancérologique, traumatologique, ORL, maxillo-faciale, parodontologique. Ce matériau peut aussi être utilisé afin de soigner des fractures avec perte de substance, et la pseudarthrose avec ou sans perte de substance. Il s'est révélé utile également pour les arthrodèses vertébrales (fusion rachidienne) et en ostéotomies d'addition. Il peut également augmenter le volume des autogreffes et en accélérer les effets.

Daculsi et al. (Rev. Chir. Orthop. 1989, 75(2), 65-71) ont décrit un matériau constitué d'hydroxyapatite et de β-TCP, (ci-après BCP) aux propriétés intéressantes

Le BCP présente une porosité globale de 70% dont 2/3 de macroporosité (300 à 600 µm) et 1/3 de microporosité (pore < 10 µm), cette dernière permettant la diffusion des fluides biologiques.

Les phénomènes de dissolution des cristaux (essentiellement le TCP) provoquent la libération d'ions dans les fluides biologiques. Cette saturation en ions conduit à une précipitation cristalline (produite en présence des propres protéines du patient) constituée de cristaux d'apatites biologiques identiques aux cristaux d'os.

Cette précipitation à court terme permet d'améliorer les propriétés mécaniques initiales, et constitue une nouvelle interface avec les cellules et les tissus qui se substitue à la surface synthétique.

Les macropores servent à guider les cellules en profondeur de l'implant (ostéo-conduction), lesquelles pourront résorber le matériau et former à la place un tissu osseux différencié.

L'os néoformé subit rapidement un remodelage osseux. Un cycle résorption-apposition se produit comme dans l'os classique. Le volume osseux augmente progressivement aux dépens du BCP. La rapide réhabilitation du BCP, grâce à l'importance de sa porosité, permet d'améliorer ses propriétés mécaniques au cours de la transformation osseuse ; l'implant BCP va acquérir les propriétés de l'os spongieux ou cortical, selon son site d'implantation.

In est important de contrôler le processus de résorption substitution osseuse, nécessaire à un véritable substitut osseux. Le BCP, par son mélange HA et β-TCP équilibré, et sa structure micro et macroporeuse, permet cette cinétique.

Une des difficultés principales rencontrées par le chirurgien utilisant des matériaux à base d'hydroxyapatite et de phosphate tricalcique, réside dans la difficulté de contrôler le succès de l'implantation du substitut osseux immédiatement après l'opération, ainsi que sa résorption et substitution au cours du temps par les tissus de l'organisme.

La radio-opacité de l'hydroxyapatite et du phosphate tricalcique β n'est en effet pas suffisamment différente de celle de l'os, et ne permet par conséquent pas de visualiser correctement le biomatériau. Il serait donc souhaitable d'inclure des agents de contraste dans les substituts osseux utilisés en chirurgie.

Les obstacles auxquels se sont heurtées la plupart des tentatives dans ce domaine sont, d'une part, la difficulté d'obtenir une répartition homogène de l'agent de contraste à l'intérieur de la matrice phosphocalcique, et d'autre part la toxicité des agents de contraste existant à ce jour, la fuite de ces derniers dans l'organisme étant proscrite.

Le document JP 2007 197329 A décrit une composition pour biomatériaux de comblement osseux, comprenant un mélange de phosphates de calcium (phosphate tetracalcique et hydrogenophosphate de calcium) avec un agent de contraste pour l'imagerie médicale. Aucune étape de frittage n'est réalisée.

Ils doivent aussi être utilisés en concentration suffisante afin d'être détectés, et néanmoins ne pas nuire à la colonisation osseuse. De plus, beaucoup d'agents de contraste, comme BaSO₄ ou ZrO₂ présentent des effets secondaires importants et peuvent induire une résorption osseuse pathologique.

L'inclusion d'agents de contraste dans ces prothèses résorbables peut également rendre celles-ci beaucoup trop dures et endommager les articulations à proximité desquelles elles sont implantées.

Les auteurs de la présente invention ont résolu les problèmes évoqués ci-dessus en obtenant, de manière surprenante, un substitut osseux comprenant du phosphate de calcium combiné à un agent de contraste pour l'imagerie médicale, qui induit une bonne repousse osseuse sans être cytotoxique.

Plus précisément, l'invention se rapporte à une composition pour biomatériau comprenant un phosphate de calcium dans lequel le rapport molaire Ca/P est compris entre 1 et 2, fritté avec un agent de contraste pour l'imagerie médicale réparti uniformément dans la masse de la composition.

On entend par « agent de contraste », au sens de la présente invention, un composé capable de créer, sur une image médicale, un contraste artificiel sur une structure anatomique ou pathologique naturellement peu ou pas contrastée, et donc difficilement différentiable des structures voisines. Cet agent de contraste est avantageusement un agent radio-opaque utile en imagerie par diffraction des rayons X, c'est-à-dire un composé qui n'est peu ou pas traversé par les rayons X. L'agent radio-opaque est avantageusement inorganique ou ionique.

On entend par « rapport molaire Ca/P », au sens de la présente invention, le rapport entre le nombre d'atomes de calcium et le nombre d'atomes de phosphore dans la formule chimique du phosphate de calcium.

L'invention permet avantageusement d'inclure des agents de contraste inorganiques ou ioniques dans la matrice de phosphate de calcium.

Le phosphate de calcium est avantageusement un mélange de phosphate tricalcique β et d'hydroxyapatite, avantageusement dans un rapport en poids phosphate tricalcique β/hydroxyapatite compris entre 20/80 et 70/30, plus avantageusement égal à 40/60.

Il est possible d'observer, dans la maille élémentaire du phosphate de calcium, la substitution partielle du calcium par le cation provenant de l'agent de contraste, et/ou la substitution partielle du phosphate ou d'un autre anion (par exemple hydroxyle, fluorure, chlorure) par l'anion de l'agent de contraste.

La proportion d'agent de contraste dans la composition selon l'invention est avantageusement comprise entre 2 et 40% du poids total, plus avantageusement entre 5 et 35%, plus avantageusement encore égale à environ 20%.

L'agent de contraste peut être choisi parmi les agents radio-opaques pour l'imagerie par diffraction des rayons X et les agents de contraste pour l'imagerie par résonance magnétique nucléaire.

L'agent de contraste est avantageusement choisi parmi le sulfate de baryum, l'oxyde de bismuth, l'oxyde de lutétium, le phosphate de gadolinium, et leurs mélanges.

Selon un mode de réalisation particulier de l'invention, la composition se présente sous forme de granule de forme irrégulière. Selon un autre mode de réalisation avantageux de l'invention, la composition se présente sous forme de granule de forme sphérique.

Avantageusement, le diamètre moyen des granules de la composition selon l'invention est compris entre 20 et 500 µm, de préférence entre 40 et 300 µm, de manière particulièrement préférée entre 80 et 200 µm.

L'invention se rapporte également à une composition selon l'invention en tant que substitut osseux.

L'invention a également pour objet une composition injectable comprenant une composition selon l'invention en suspension dans un hydrogel.

L'hydrogel comprend avantageusement une solution aqueuse d'un dérivé de la cellulose, avantageusement l'hydroxypropylméthylcellulose.

L'invention se rapporte également à une composition selon l'invention ou à une composition injectable selon l'invention en tant que substitut osseux.

La composition selon l'invention pourra avantageusement être utilisée comme matériau de comblement osseux dans toute chirurgie de reconstruction ou de régénération osseuse. La composition selon l'invention pourra également être utilisée dans des implants et ostéosynthèses à base de polymères et de granules, comme par exemple les polymères résorbables de la famille des PLA-GLA (acide polylactique-acide polyglycolique), ou les polymères non résorbables comme le PEEK (polyéther éther cétone).

La composition injectable selon l'invention pourra avantageusement être utilisée comme matériau de comblement dans toute chirurgie de reconstruction ou de régénération tissulaire osseuse, par exemple articulaire et dentaire, et notamment en chirurgie invasive a *minima* (« minimally invasive surgery »)

L'invention se rapporte également à un procédé de préparation d'une composition selon l'invention, caractérisée en ce qu'elle comprend les étapes successives suivantes :
(a) mélanger un phosphate de calcium dans lequel le rapport molaire Ca/P est compris entre 1 et 2, avantageusement de l'apatite déficiente en calcium, avec un agent de contraste jusqu'à obtenir une répartition uniforme de l'agent de contraste dans le phosphate de calcium ;
(b) granuler le mélange résultant ;
(c) fritter les granules à une température supérieure à 600°C, avantageusement à une température comprise entre 700 et 1300°C ;
(d) éventuellement stériliser les granules frittés.

L'invention a donc également pour objet une composition susceptible d'être obtenue selon le procédé ci-dessus.

On entend par « apatite déficiente en calcium », au sens de la présente invention, une apatite non stoechiométrique. Celle-ci peut avantageusement obéir à la formule Caₓ (PO₄)_{y} (X)₁₋₂, dans laquelle X peut être choisi parmi OH, F et Cl, et où x est compris entre 7 et 10 exclu, et y entre 3 et 6 exclu. Elle peut également avoir pour formule Ca₉(HPO₄)(PO₄)₅OH.

On entend par « fritter », au sens de la présente invention, l'action de consolider par action de la chaleur un agglomérat granulaire plus ou moins compact, avec ou sans fusion d'un ou de plusieurs de ses constituants.

Différentes techniques de granulation peuvent être utilisées pour obtenir ces granules comprenant un agent de contraste.

Le phosphate de calcium, avantageusement une apatite déficiente en calcium, peut être est préparé par réaction entre un sel de calcium (par exemple le nitrate de calcium ou l'acétate de calcium) et un sel de phosphate (par exemple le phosphate d'ammonium ou le phosphate de sodium) dans lesquels le rapport molaire Ca/P est compris entre 1 et 2. Elle est suivie d'une précipitation en milieu aqueux basique à une température de 25 à 80°C, puis d'une phase de maturation basique, qui permet d'augmenter la cristallinité et de contrôler et augmenter le ratio Ca/P de l'apatite finale.

Une autre voie de synthèse du phosphate de calcium peut mettre en oeuvre la réaction entre l'hydroxyde de calcium avec une solution d'acide phosphorique.

La première mise en forme (étape a) consiste à obtenir une répartition de l'agent de contraste dans le phosphate de calcium. Cette homogénéisation peut se faire en milieu aqueux ou sous forme sèche, avantageusement en milieu aqueux.

Il est particulièrement avantageux d'obtenir un mélange homogène, dans lequel l'agent de contraste est réparti de façon uniforme au sein de la matrice phosphocalcique, avant tout traitement thermique.

La poudre, comprenant le phosphate de calcium et l'agent de contraste, peut tout d'abord être broyée au broyeur centrifugeur, et dispersée pour éviter les agglomérats. Le mélange homogène est ensuite avantageusement obtenu par l'utilisation d'un mélangeur à trois dimensions (dit « turbulat ») pour les formes sèches, ou par mélangeur rotatif pour les formes humides. La poudre obtenue a avantageusement une taille inférieure à 20 µm.

Une fois le mélange homogène obtenu, la granulation de ce produit peut être réalisée (étape b), par des techniques classiques connues de l'homme du métier.

Deux voies principales peuvent avantageusement être exploitées pour obtenir les granules selon l'invention : la voie humide et la voie sèche, selon des techniques connues de l'homme du métier et abondamment décrites.

La voie humide peut consister essentiellement à utiliser l'eau comme liant avec la poudre obtenue à l'étape (a). On pourra citer à titre d'exemple de techniques de granulation humide convenant à la réalisation de l'invention : le fractionnement mécanique (utilisation d'un granulateur humide, la matière humide étant forcée à travers un tamis) ; le séchage direct dans un flux d'air chaud (utilisation d'un « spray dryer », la matière étant dispersée dans un liquide et injectée dans un flux d'air plus ou moins chaud) ; la technique de lit d'air fluidisé ; la technique de l'assiette granulatrice ; le tambour granulateur ; la dispersion de gouttes (« prilling »).

La voie sèche peut consister à agglomérer des particules très fines par un mouvement mécanique spécifique. Grâce à la formation de liaisons chimiques, électrostatiques et/ou magnétiques, et/ou à l'addition d'un liant, et/ou encore à cause de leur forme, les particules auront tendance à s'imbriquer entre elles. Dans ce cas, la technique de compaction (presse), suivie de la technique de fractionnement par broyage peuvent être utilisées (au moyen par exemple d'une assiette granulatrice, d'un tambour granulateur, sans addition de liant).

Une fois les granules obtenus, un frittage à haute température, avantageusement supérieure à 600°C, plus avantageusement comprise entre 700 et 1300°C, est réalisé, afin de décomposer le phosphate de calcium comprenant l'agent de contraste en hydroxyapatite et en phosphate tricalcique β comprenant l'agent de contraste. Cette dernière étape peut avantageusement permettre d'intégrer un élément radio opaque, comme par exemple le baryum, dans la maille élémentaire du phosphate de calcium.

Le frittage peut être effectué selon des techniques bien connues de l'homme du métier. Il permet également d'obtenir une bonne tenue mécanique et un matériau de type cristallin.

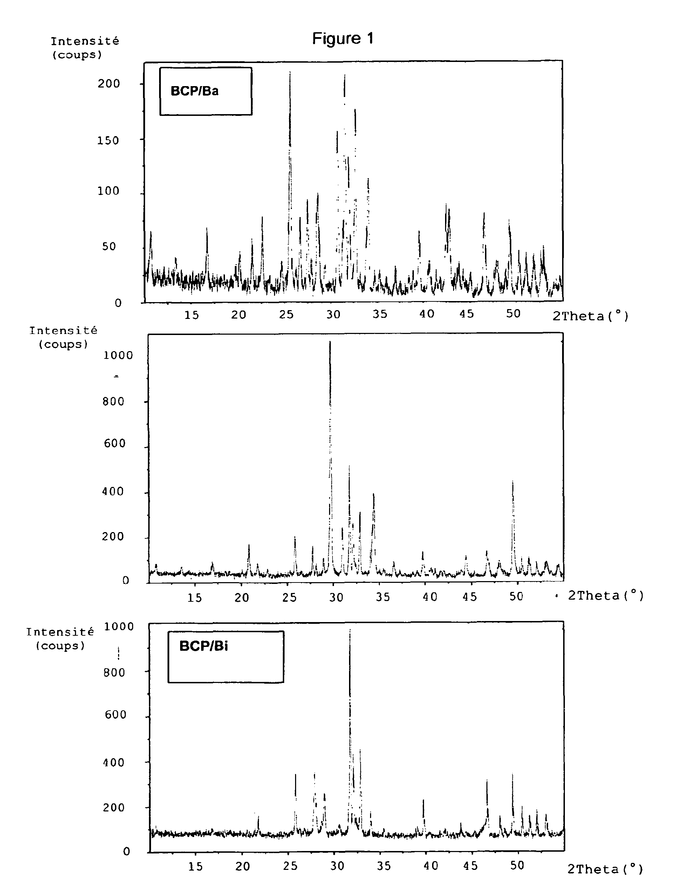

L'invention va maintenant être illustrée de façon non limitative par les exemples 1 à 4 et les figures 1 à 5 suivantes.
La figure 1 représente les spectres de diffraction des rayons X de compositions selon l'invention.
La figure 2A représente la surface de granules selon l'invention observée par microscopie électronique à balayage, et la figure 2B cette même surface après quatre jours de culture de cellules MCT3-E1.
La figure 3 représente des radiographies de l'épiphyse fémorale de rats dans laquelle a été implanté un granule radio opaque, après trois semaines d'implantation.
La figure 4A illustre une image obtenue par microscopie électronique à balayage de l'épiphyse fémorale de rats dans laquelle a été implanté un granule radio opaque juste après l'implantation. La figure 4B correspond à un traitement de l'image de cette épiphyse après 3 semaines d'implantation, afin de visualiser l'os nouvellement formé en blanc.
La figure 5 représente une image au microscope à lumière polarisée montrant la repousse osseuse et la colonisation tissulaire chez le rat après 3 semaines d'implantation.

### Exemple 1 : préparation de substituts osseux comprenant un agent de contraste

Quatre composés radio opaques ont été testés : le sulfate de baryum (BaSO₄), l'oxyde de lutétium (Lu₂O₃), l'oxyde de bismuth (Bi₂O₃) et le phosphate de gadolinium (GdPO₄). Chacun de ces composés a été mélangé à 20% en poids à de l'apatite déficiente en calcium. Le matériau composite a été préparé en granules ronds de diamètre moyen compris entre 80 et 200 µm. Les granules ont été frittés à 1050°C, pour décomposer l'apatite déficiente en calcium en hydroxyapatite et β-TCP dans un rapport en poids hydroxyapatite/β-TCP de 60/40, puis ils ont été stérilisés à la vapeur (121°C, 30 minutes) ou à sec (180°C, quatre heures) pour être utilisés dans les études *in vitro* et *in vivo.*

Ces granules seront par la suite dénommés BCP/Ba, BCP/Lu, BCP/Bi et BCP/Gd.

Les diffractogrammes des BCP/Ba, BCP/Lu, BCP/Bi montrent des modifications des paramètres cristallographiques a et c de l'hydroxyapatite et du β-TCP, attribués à l'intégration de cations et d'anions de l'agent de contraste dans la maille du phosphate de calcium. Ce phénomène est le plus marqué dans le cas du baryum.

### Exemple 2 : biocompatibilité in vitro

La biocompatibilité des substituts osseux préparés à l'exemple ci-dessus a été évaluée selon la norme ISO 10993-5 sur des extraits de cellules ostéoblastiques MC3T3-E1. Les matériaux ont dans ce cas été préparés, non sous la forme de granules, mais de pastilles de 10 mm de diamètre et 1 mm d'épaisseur, par compactage uniaxial. La cytotoxicité a été mesurée en utilisant un test MTS. Les résultats sont rassemblés dans le tableau 1 ci-dessous.

**Tableau 1 : activité MTS des cellules MC3T3-E1 après 72 h de culture, selon la norme ISO 10993-5.**

| **Matériaux** | Plastique (contrôle) | Act D | BCP/Ba | BCP/Bi | BCP/Lu |
|---|---|---|---|---|---|
| **Activité des cellules MTS à 72 h (%)** | 100 | 10 | 100 | 98 | 55 |

Aucune cytotoxicité n'a été détectée pour BCP/Ba et BCP/Bi, alors que BCP/Lu s'est révélé cytotoxique pour les cellules ostéoblastiques (décroissance de l'activité MTS de plus de 20%). Act D est l'abréviation d'actynomycine D à 5µg/ml (contrôle négatif).

### Exemple 3 : microscopie électronique à balayage

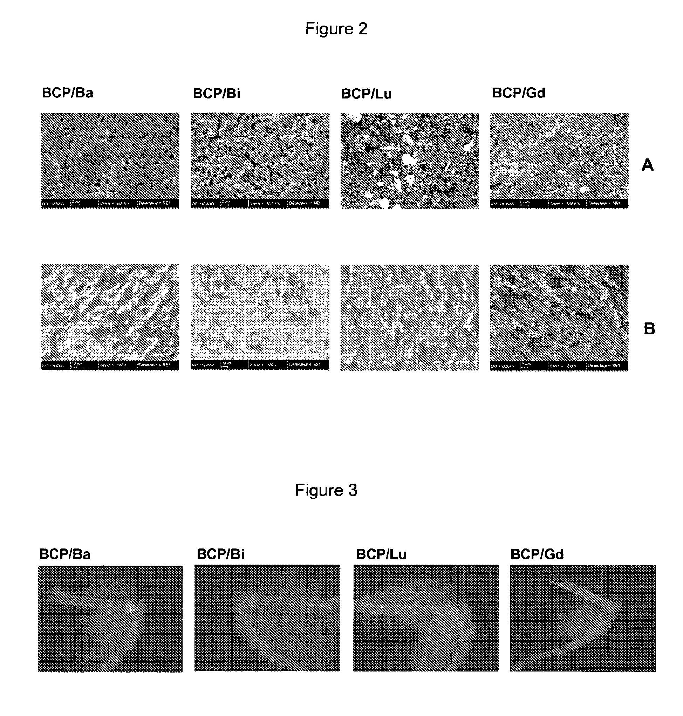

L'observation par microscopie électronique à balayage des surfaces composites (figure 2A), montre que l'addition des agents de contraste à la matrice de phosphate de calcium a une influence sur la forme du cristal, sur sa taille et sur sa microporosité. Ainsi, l'addition de BaSO₄ a conduit à la formation de gros cristaux ayant une faible microporosité et une surface lisse.

L'addition de Bi₂O₃ a conduit à la croissance de cristaux en aiguille ayant une microporosité importante dans le cas de Lu₂O₃ et GdPO₄, la microporosité est importante et les cristaux sont de petite taille. De nombreux fragments sont présents sur la matrice BCP/Lu₂O₃.

La morphologie des cellules MC3T3-E1 a été déterminée au quatrième jour de culture en contact direct avec les granules. Après fixation sur les cellules, les granules ont été recouverts d'or et de palladium et observés au microscope électronique à balayage, par diffraction d'électrons rétrodiffusés.

Les images obtenues sont reproduites à la figure 2B.

Le type de la matrice a une influence sur l'évolution des cellules. Les cellules MC3T3-E1 ont une morphologie ostéoblastique classique dans tous les cas (pseudopodia longue avec un bon étalement), sauf pour la matrice comportant Bi₂O₃ pour laquelle la morphologie et l'étalement des cellules se sont trouvés modifiés.

La prolifération de cellules s'est en revanche trouvée grandement affectée par le type de granules. La prolifération des cellules a été plus importante sur BCP/Ba, moins importante et à peu près similaire dans le cas de BCP/Lu et BCP/Gd et encore plus faible sur BCP/Bi.

### Exemple 4 : implantation animale et histologie

Les granules ont été implantés dans l'épiphyse fémorale de deux rats femelle de type Wistar. Un défaut de 3 mm de diamètre et de 5 mm a été effectué et comblé avec les granules. Après 3 semaines d'implantation, des radiographies ont été enregistrées afin d'évaluer la radio-opacité.

Les images obtenues sont reproduites à la figure 3.

L'évaluation du pouvoir radio-opaque *in situ* des différents matériaux a montré que le BCP/Ba et le BCP/Bi présentent le meilleur contraste par rapport à l'environnement osseux.

Ensuite, les implants ont été prélevés, fixés dans une solution formolée et inclus dans de la résine de méthacrylate de glycol. Les blocs ont été découpés en coupes de 30 à 100 µm d'épaisseur au microtome à scie diamantée, et analysés en microscopie photonique et en microscopie électronique à balayage.

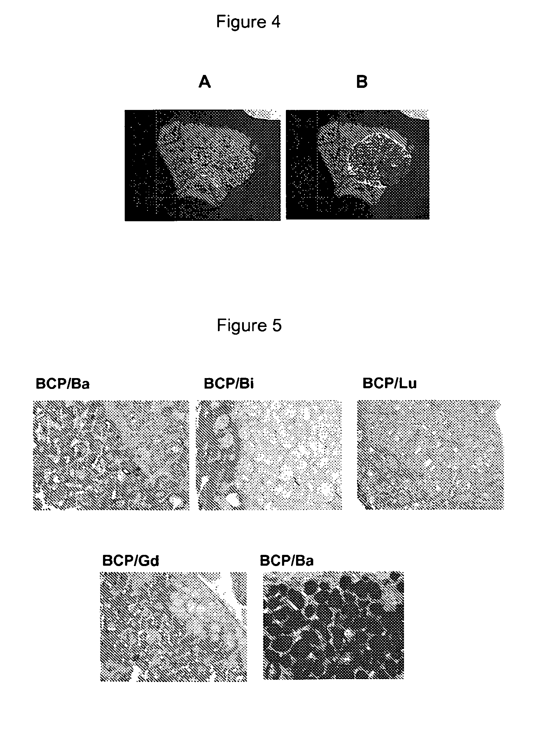

Les radiographies obtenues sont reproduites aux figures 4 et 5. Une quantification de la colonisation osseuse, réalisée par analyse d'image automatisée en 2D en microscopie électronique à balayage (électrons rétrodiffusés, BSE) est donnée dans le tableau 2 ci-dessous.

**Tableau 2 : quantification de la colonisation osseuse dans le matériau**

| **Matériau** | **Surface totale (%)** | **Surface du biomatériau (%)** | **Surface de l'os néoformé (%)** |
|---|---|---|---|
| BCP/Ba | 100 | 50 | 15 |
| BCP/Bi | 100 | 45 | 7 |
| BCP/Lu | 100 | 44 | 7 |
| BCP/Gd | 100 | 37 | 13 |

Les résultats histologiques complémentaires présentés aux figures 4 et 5 permettent d'évaluer la quantité d'os nouvellement formé et la résorption des granules (figure 4), ainsi que la qualité de la repousse osseuse en contact avec le matériau (figure 5). Les granules BCP/Ba et BCP/Gd présentent la repousse osseuse la meilleure d'un point de vue quantitatif et qualitatif par rapport à BCP/Bi et BCP/Lu.

## Revendications

1. Composition pour biomatériaux, **caractérisée en ce qu'**elle comprend un phosphate de calcium dans lequel le rapport molaire Ca/P est compris entre 1 et 2, constitué de phosphate tricalcique β et d'hydroxyapatite dans un rapport en poids phosphate tricalcique β/hydroxyapatite compris entre 20/80 et 70/30, fritté avec un agent de contraste pour l'imagerie médicale réparti uniformément dans la masse de la composition.

2. Composition selon la revendication 1, **caractérisée en ce que** l'agent de contraste est un composé radio-opaque, de préférence un composé inorganique.

3. Composition selon l'une quelconque des revendications précédentes, **caractérisée en ce que** le rapport en poids phosphate tricalcique β/hydroxyapatite est égal à 40/60.

4. Composition selon l'une quelconque des revendications précédentes, **caractérisée en ce que** la proportion d'agent de contraste est comprise entre 2 et 40% du poids total, de préférence entre 5 et 35%, plus préférentiellement encore égale à environ 20%.

5. Composition selon l'une quelconque des revendications précédentes, **caractérisée en ce que** l'agent de contraste est choisi parmi les agents radio-opaques pour l'imagerie par diffraction des rayons X et les agents de contraste pour l'imagerie par résonance magnétique nucléaire.

6. Composition selon l'une quelconque des revendications précédentes, **caractérisée en ce que** l'agent de contraste est choisi parmi le sulfate de baryum, l'oxyde de bismuth, l'oxyde de lutétium, le phosphate de gadolinium, et leurs mélanges.

7. Composition selon l'une quelconque des revendications précédentes, **caractérisée en ce qu'**elle se présente sous forme de granules sphériques.

8. Composition selon l'une quelconque des revendications précédentes, **caractérisée en ce que** le diamètre moyen des granules est compris entre 20 et 500 µm, de préférence entre 80 et 200 µm.

9. Composition injectable comprenant une composition selon l'une quelconque des revendications précédentes en suspension dans un hydrogel.

10. Composition injectable selon la revendication 9, **caractérisée en ce que** l'hydrogel comprend une solution aqueuse d'un dérivé de la cellulose, de préférence l'hydroxypropylméthylcellulose.

11. Composition selon l'une quelconque des revendications précédentes en tant que substitut osseux.

12. Procédé de préparation d'une composition selon l'une quelconque des revendications 1 à 8, **caractérisée en ce qu'**elle comprend les étapes successives suivantes :
(a) mélanger un phosphate de calcium dans lequel le rapport molaire Ca/P est compris entre 1 et 2, de préférence de l'apatite déficiente en calcium, avec un agent de contraste jusqu'à obtenir une répartition uniforme de l'agent de contraste dans le phosphate de calcium ;
(b) granuler le mélange résultant ;
(c) fritter les granules, à une température supérieure à 600°C, de préférence à une température comprise entre 700 et 1300°C ;
(d) éventuellement stériliser les granules frittés.

## Claims

1. A composition for biomaterials, **characterized in that** it comprises calcium phosphate in which the molar ratio Ca/P is comprised between 1 and 2, consisting of β tricalcium phosphate and of hydroxyapatite in a β tricalcium phosphate/hydroxyapatite weight ratio comprised between 20/80 and 70/30, sintered with a contrast agent for medical imaging, uniformly distributed in the bulk of the composition.

2. The composition according to claim 1, **characterized in that** the contrast agent is a radio-opaque compound, preferably an inorganic compound.

3. The composition according to any of the preceding claims, **characterized in that** the β tricalcium phosphate/hydroxyapatite weight ratio is equal to 40/60.

4. The composition according to any of the preceding claims, **characterized in that** the proportion of contrast agent is comprised between 2 and 40% of the total weight, preferably between 5 and 35%, even more preferentially equal to about 20%.

5. The composition according to any of the preceding claims, **characterized in that** the contrast agent is selected from radio-opaque agents for X-ray diffraction imaging and contrast agents for nuclear magnetic resonance imaging.

6. The composition according to any of the preceding claims, **characterized in that** the contrast agent is selected from barium sulfate, bismuth oxide, lutetium oxide, gadolinium phosphate, and mixtures thereof.

7. The composition according to any of the preceding claims, **characterized in that** it appears as spherical granules.

8. The composition according to any of the preceding claims, **characterized in that** the average diameter of the granules is comprised between 20 and 500 µm, preferably between 80 and 200 µm.

9. An injectable composition comprising a composition according to any of the preceding claims, suspended in a hydrogel.

10. The injectable composition according to claim 9, **characterized in that** the hydrogel comprises an aqueous solution of a derivative of cellulose, preferably hydroxypropylmethyl cellulose.

11. The composition according to any of the preceding claims as a bone substitute.

12. A method for preparing a composition according to any of claims 1 to 8, **characterized in that** it comprises the following successive steps:
(a) mixing calcium phosphate in which the molar ratio Ca/P is comprised between 1 and 2, preferably calcium-deficient apatite, with a contrast agent until a uniform distribution of the contrast agent is obtained in the calcium phosphate;
(b) granulating the resulting mixture;
(c) sintering the granules at a temperature above 600°C, preferably at a temperature comprised between 700 and 1,300°C;
(d) optionally sterilizing the sintered granules.

## Patentansprüche

1. Zusammensetzung für Biomaterialien, **dadurch gekennzeichnet, dass** sie ein Calciumphosphat umfasst, in dem das Molverhältnis Ca/P 1 bis 2 beträgt, das aus β-Tricalciumphosphat und Hydroxyapatit in einem Gewichtsverhältnis β-Tricalciumphosphat/Hydroxyapatit von 20/80 bis 70/30 besteht, und das mit einem Kontrastmittel für bildgebende Verfahren in der Medizin, welches gleichmäßig in der Masse der Zusammensetzung verteilt ist, gesintert ist.

2. Zusammensetzung gemäß Anspruch 1, **dadurch gekennzeichnet, dass** das Kontrastmittel eine strahlenundurchlässige Verbindung, bevorzugt eine anorganische Verbindung, ist.

3. Zusammensetzung gemäß irgendeinem der vorhergehenden Ansprüche, **dadurch gekennzeichnet, dass** das Gewichtsverhältnis β-Tricalciumphosphat/Hydroxyapatit 40/60 beträgt.

4. Zusammensetzung gemäß irgendeinem der vorhergehenden Ansprüche, **dadurch gekennzeichnet, dass** der Anteil an Kontrastmittel 2 bis 40 %, bevorzugt 5 bis 35 %, noch bevorzugter etwa 20 %, des Gesamtgewichts ausmacht.

5. Zusammensetzung gemäß irgendeinem der vorhergehenden Ansprüche, **dadurch gekennzeichnet, dass** das Kontrastmittel ausgewählt ist aus strahlenundurchlässigen Mitteln zur Bildgebung durch Röntgenstrahldiffraktion und Kontrastmitteln zur Bildgebung durch Kernspinresonanz.

6. Zusammensetzung gemäß irgendeinem der vorhergehenden Ansprüche, **dadurch gekennzeichnet, dass** das Kontrastmittel ausgewählt ist aus Bariumsulfat, Wismutoxid, Lutetiumoxid, Gadoliniumphosphat und deren Mischungen.

7. Zusammensetzung gemäß irgendeinem der vorhergehenden Ansprüche, **dadurch gekennzeichnet, dass** sie in Form sphärischer Körnchen vorliegt.

8. Zusammensetzung gemäß irgendeinem der vorhergehenden Ansprüche, **dadurch gekennzeichnet, dass** der mittlere Durchmesser der Körnchen 20 bis 500 µm, bevorzugt 80 bis 200 µm, beträgt.

9. Injizierbare Zusammensetzung, welche eine Zusammensetzung gemäß irgendeinem der vorhergehenden Ansprüche als Suspension in einem Hydrogel umfasst.

10. Injizierbare Zusammensetzung gemäß Anspruch 9, **dadurch gekennzeichnet, dass** das Hydrogel eine wässrige Lösung eines Zellulosederivats, bevorzugt Hydroxypropylmethylzellulose, umfasst.

11. Zusammensetzung gemäß irgendeinem der vorhergehenden Ansprüche als Knochenersatz.

12. Verfahren zu Herstellung einer Zusammensetzung gemäß irgendeinem der Ansprüche 1 bis 8, **dadurch gekennzeichnet, dass** es die folgenden Schritte umfasst:
(a) Mischen eines Calciumphosphats, in dem das Molverhältnis Ca/P 1 bis 2 beträgt, bevorzugt calciumarmes Apatit, mit einem Kontrastmittel bis zum Erhalt einer gleichmäßigen Verteilung des Kontrastmittels in dem Calciumphosphat;
(b) Granulieren der resultierenden Mischung;
(c) Sintern der Körnchen bei einer Temperatur von über 600 °C, bevorzugt bei einer Temperatur von 700 bis 1300 °C;
(d) gegebenenfalls Sterilisieren der gesinterten Körnchen.
